# EUROPÄISCHE PATENTANMELDUNG

(11) **EP 1 228 764 A1**
(43) Veröffentlichungstag der Anmeldung: **07.08.2002**
(21) Anmeldenummer: 01810101.4
(22) Anmeldetag: 01.02.2001
(51) Int. Cl.: A61K 33/06, A61K 31/702

(54) **Calciumkomposition mit Oligofructose**

(71) Anmelder: EnergyBalance AG, 9491 Ruggell (LI)
(72) Erfinder:

(57) **Zusammenfassung**

Die intestinale Calciumabsorption kann durch Oligofructose erhöht werden. Somit kann die therapeutische Wirkung einer Calciumsupplementierung durch die Einnahme von Oligofructose verbessert werden.

## Beschreibung

Die intestinale Calciumabsorption kann durch Oligofructose erhöht werden. Somit kann die therapeutische Wirkung einer Calciumsupplementierung durch die Einnahme von Oligofructose verbessert werden.

## Patentansprüche

1. Kompositionen mit Calcium für die orale Anwendung bei Mensch und Tier
**dadurch gekennzeichnet,**
**dass** sie Oligofructose enthalten.

2. Vitamin- und Mineralkompositionen mit Calcium für die orale Anwendung bei Mensch und Tier
**dadurch gekennzeichnet,**
**dass** sie Oligofructose enthalten.

## Geänderte Patentansprüche

### Geänderte Patentansprüche gemäss Regel 86(2) EPÜ.

**1.** Alle Kompositionen, die nebst Calcium weitere Zutaten enthalten, ausgenommen Reformlebensmittel mit Milchprodukten, für die orale Anwendung bei Menschen bestimmt sind und welche nicht für die Behandlung und Prävention von Osteoporose, nicht als Calciumsupplement und nicht gegen Calciummangel deklariert oder bestimmt sind, **dadurch gekennzeichnet,**
**dass** sie Präbiotika enthalten.

**2.** Alle Kompositionen, die Calcium enthalten und für die orale Anwendung bei Menschen bestimmt sind, ausgenommen Reformlebensmittel mit Milchprodukten, und welche nicht für die Behandlung und Prävention von Osteoporose,
nicht als Calciumsupplement und nicht gegen Calciummangel deklariert oder bestimmt sind, **dadurch gekennzeichnet,**
**dass** sie Präbiotika enthalten.
